(19) Europäisches Patentamt
European Patent Office
Office européen des brevets

(11) EP 2 987 852 A1

(12) EUROPEAN PATENT APPLICATION
published in accordance with Art. 153(4) EPC

(43) Date of publication:
**24.02.2016 Bulletin 2016/08**

(21) Application number: **14785390.7**

(22) Date of filing: **16.04.2014**

(51) Int Cl.:
***C12N 1/00*** *(2006.01)* ***C12N 5/0789*** *(2010.01)*

(86) International application number:
**PCT/JP2014/060829**

(87) International publication number:
**WO 2014/171486 (23.10.2014 Gazette 2014/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.04.2013 JP 2013086904**

(71) Applicant: **Nissan Chemical Industries, Ltd.**
**Chiyoda-ku**
**Tokyo 101-0054 (JP)**

(72) Inventors:
- **NISHINO, Taito**
  **Shiraoka-shi**
  **Saitama 349-0294 (JP)**
- **KANAKI, Tatsuro**
  **Shiraoka-shi**
  **Saitama 349-0294 (JP)**
- **OTANI, Ayako**
  **Shiraoka-shi**
  **Saitama 349-0294 (JP)**
- **SARUHASHI, Koichiro**
  **Funabashi-shi**
  **Chiba 274-8507 (JP)**
- **TOMURA, Misayo**
  **Funabashi-shi**
  **Chiba 274-8507 (JP)**

(74) Representative: **von Kreisler Selting Werner - Partnerschaft**
**von Patentanwälten und Rechtsanwälten mbB**
**Deichmannhaus am Dom**
**Bahnhofsvorplatz 1**
**50667 Köln (DE)**

# (54) MEDIUM COMPOSITION AND METHOD FOR PRODUCING RED BLOOD CELLS USING SAME

(57) The present invention provides a method of producing erythrocytes, including efficiently differentiating hematopoietic stem cells and/or a hematopoietic progenitor cells into erythrocytes by using a medium composition having an effect of homogeneously dispersing the hematopoietic stem cells and/or the hematopoietic progenitor cells and maintaining a floating state.



Printed by Jouve, 75001 PARIS (FR)

## Description

Technical Field

The present invention relates to a medium composition containing polysaccharides, and an ex vivo production method of erythrocytes comprising using the medium composition.

Background Art

Transfusion of erythrocytes is used as a treatment method of anemia for which a medicament therapy is ineffective, and bleeding due to trauma or surgery. While the supply thereof mainly relies on spontaneous blood donation, works for collection, test and preservation for stable reservation of the blood require a large amount of labor. Erythrocyte preparations derived from donated blood cannot completely eliminate infection with a virus such as HIV and hepatitis virus, and unknown infection sometimes cannot be detected. To stably supply safe erythrocytes in such situation, the need for ex vivo production of erythrocytes as an alternative to donated blood is increasing (non-patent document 1).

As for in vivo differentiation of erythrocytes, it is known that erythrocyte is produced starting from hematopoietic stem cell and via erythrocyte/megakaryocyte progenitor cell, erythroid progenitor cell (BFU-E, CFU-E), proerythroblast, basophilic erythroblast, polychromatophil erythroblast, orthochromic erythroblast, and reticulocyte. Also, as a major factor that promotes erythrocyte differentiation, erythropoietin (EPO) and stem cell factor (SCF) have been reported (non-patent documents 2, 3). Based on these findings, ex vivo methods for reproducing erythrocyte differentiation and producing erythrocytes have been developed.

For example, there have been developed methods of inducing erythrocytes from ES cells or iPS cells, which are pluripotent stem cells (patent document 1, non-patent documents 4, 5), and methods of inducing erythrocytes from CD34 positive cells derived from peripheral blood, fetal liver, bone marrow or cord blood (non-patent documents 3, 6). In addition, a technique for establishing an erythrocyte progenitor cell line from pluripotent stem cells and preparing a large number of erythrocytes from the erythrocyte progenitor cells has also been studied (non-patent document 7). However, these culture methods cannot easily produce erythrocytes in a short time at high efficiency, and particularly have a problem of improving efficiency of the enucleation process (patent document 2). Furthermore, a fear of canceration by transfusion of nucleated erythrocyte progenitor cells also poses a problem in ex vivo expansion of erythrocytes.

[Document List]

[patent documents]

patent document 1: WO 2009/137629
patent document 2: WO 2010/098079

[non-patent documents]

non-patent document 1: Mountford et al., British Journal of Haematology 2010, 149:22-34
non-patent document 2: Dolznig et al., Curr. Biol. 2002, 12:1076-1085
non-patent document 3: Giarratana et al., Blood 2011, 118:5071-5079
non-patent document 4: Ma et al., Proceedings of the National Academy of Sciences 2008, 105:13087-13092
non-patent document 5: Dias et al., Stem Cells and Development 2011, 20:1639-1647
non-patent document 6: Fujimi et al., International Journal of Hematology 2008, 87:339-350
non-patent document 7: Hiroyama et al., PLoS ONE 2008, 3:e1544

SUMMARY OF THE INVENTION

Problems to be Solved by the Invention

An object of the present invention is to solve the above-mentioned problem of the prior art, and provide a medium composition for producing erythrocytes ex vivo in a short time at high efficiency and a production method of erythrocytes using the composition.

Means of Solving the Problems

The present inventors have conducted intensive studies on the effect of various compounds and liquid media containing them on erythrocyte differentiation and successfully found a medium composition that promotes differentiation of erythrocyte. Furthermore, they have found that, by the use of the medium composition, differentiation of erythrocyte progenitor cell into erythrocyte can be induced, and erythrocytes can be efficiently produced ex vivo.

That is, the present invention is as follows:

(1) A medium additive comprising a polysaccharide, which is used for differentiating hematopoietic stem cells and/or hematopoietic progenitor cells into erythrocytes.
(2) The additive of (1), wherein said polysaccharide has an anionic functional group.
(3) The additive of (2), wherein said anionic functional group is at least one selected from the group consisting of carboxyl group, sulfo group and phosphate group.
(4) The additive of (3), wherein said polysaccharide is at least one selected from the group consisting of hyaluronic acid, gellan gum, deacylated gellan gum, xanthan gum, carageenan, diutan gum, alginic acid, fucoidan, pectin, pectic acid, pectinic acid, heparan sulfate, heparin, heparitin sulfate, keratosulfate, chondroitin sulfate, dermatan sulfate, rhamnan sulfate and salts thereof.
(5) The additive of (4), wherein said polysaccharide is deacylated gellan gum or a salt thereof.
(6) A medium composition for erythrocyte differentiation, which comprises the additive of any of (1) to (5).
(7) The medium composition of (6), further comprising one or two or more factors selected from the group consisting of SCF, IL-3, IL-6, IL-11, FL, TPO and EPO.
(8) A method of producing erythrocytes from hematopoietic stem cells and/or hematopoietic progenitor cells, comprising culturing the hematopoietic stem cells and/or the hematopoietic progenitor cells in the presence of the additive of any of (1) to (5), or in the medium composition of (6) or (7).
(9) A method of inducing differentiation of hematopoietic stem cells into erythrocytes or progenitor cells thereof, comprising culturing the hematopoietic stem cells in the presence of the additive of any of (1) to (5), or in the medium composition of (6) or (7).
(10) A culture preparation of hematopoietic stem cells and/or hematopoietic progenitor cells, comprising hematopoietic stem cells and/or hematopoietic progenitor cells, and the medium composition of (6) or (7).

Effect of the Invention

The present invention provides a medium composition containing a particular compound (hereinafter to be also referred to as "a particular compound"), particularly a polymer compound (polysaccharide etc.) having an anionic functional group. Using the medium composition, differentiation into erythrocyte is induced, or differentiation into erythrocyte is promoted, which enables efficient ex vivo production of erythrocytes. Since the present invention provides a method of obtaining a large number of erythrocytes ex vivo in a short time, it can be preferably utilized for the treatment of a disease or injury requiring transfusion of erythrocytes. Description of Embodiments

The present invention is explained in more detail in the following.

The terms used in the present specification are defined as follows.

The hematopoietic stem cell in the present invention is a cell having multipotency permitting differentiation into any blood cell differentiation lineage of hemocyte, and also capable of self-replicating while maintaining the multipotency. The hematopoietic progenitor cell is a cell population including both a pluripotent hematopoietic progenitor cell that can differentiate into plural blood cell differentiation lineages and a unipotent hematopoietic progenitor cell that can differentiate into a single blood cell differentiation lineage. The erythrocyte progenitor cell is a hematopoietic progenitor cell that can only be differentiated into a blood cell of the erythrocyte lineage unidirectionally, and includes erythrocyte/megakaryocyte progenitor cell, burst-forming unit-erythroid (BFU-E), colony-forming unit-erythroid (CFU-E), proerythroblast, basophilic erythroblast, polychromatophil erythroblast, orthochromic erythroblast, and reticulocyte. The hematopoietic stem cell, hematopoietic progenitor cell and erythrocyte progenitor cell may be collected from bone marrow, cord blood, spleen, fetal liver or peripheral blood, or may be obtained by ex vivo induction of differentiation from pluripotent stem cells such as iPS cells (induced pluripotent stem cells), ES cells (embryonic stem cells) and the like can also be used. These cells may be purchased from reagent companies such as Takara Bio Inc., Lonza Japan, Veritas Ltd., and the like. The derivation of hematopoietic stem cell, hematopoietic progenitor cell and erythrocyte progenitor cell is not particularly limited as long as they are derived from mammals. Preferred are, for example, human, dog, cat, mouse, rat, rabbit, swine, bovine, horse, sheep, goat and the like, and more preferred is human.

CD34 positive means that CD (cluster of differentiation) 34 antigen is expressed on a cellular surface. This antigen is a marker of hematopoietic stem cells and hematopoietic progenitor cells, and disappears as the differentiation proceeds. CD34 positive cells are a cell population containing many hematopoietic stem cells and hematopoietic progenitor cells, and can be preferably used for the production of erythrocytes in the present invention. As a similar cell population, CD133 positive cells can also be mentioned.

Pluripotent stem cell is a cell simultaneously having differentiation pluripotency permitting differentiation into many kinds of cells constituting living organisms, such as endoderm (e.g., inner gastric wall, gastrointestinal tract and lung), mesoderm (e.g., muscle, bone, blood and urogenital system) and ectoderm (e.g., epidermis tissue and nerve system) lineage cells and the like, and self-replication competence enabling maintenance of differentiation pluripotency even after division and growth. Examples thereof include ES cell, iPS cell, embryonic germ cell (EG cell), Muse cell and the like. ES cell refers to a pluripotent stem cell derived from an embryo in the blastocyst stage which is an early developmental stage of animal. iPS cell is also called an artificial pluripotent stem cell or an induced pluripotent stem cell, and is a cell that acquired differentiation pluripotency and self-replication competence equivalent to those of ES cell by introducing several kinds of transcription factor genes into somatic cells such as fibroblast and the like. EG cell is a pluripotent stem cell derived from spermatogonium (reference document: Nature. 2008, 456, 344-349).

The pluripotent stem cell to be used in the present invention may be any pluripotent stem cell as long as it simultaneously has differentiation pluripotency and self-replication competence, and can differentiate into an erythrocyte. Preferable examples of the pluripotent stem cell include ES cell, iPS cell, embryonic germ cell (EG cell), Muse cell and the like, and more preferred are ES cell and iPS cell. As a transcription factor gene necessary for acquiring differentiation pluripotency in establishing iPS cells, Nanog, Oct3/4, Sox2, Sox1, Sox3, Sox15, Sox17, Klf4, c-Myc, N-Myc, L-Myc, Lin28, ERas and the like are known. These reprogramming factors may be used in any combination. Introduction of genes selected from these genes, for example, a combination of Oct3/4, Sox2, Klf4 and c-Myc, a combination of Oct3/4, Sox2, Nanog and Lin28, or a combination of Oct3/4, Sox2 and Klf4 into somatic cells such as fibroblasts and the like enables establishment of iPS cells. The iPS cell to be used in the present invention does not require a specific establishing method, and may be an iPS cell obtained by an establishing method including introduction of genes different from the above-mentioned genes, or an establishing method using a protein, a low-molecular-weight compound (histone deacetylase (HDAC) inhibitor, MEK inhibitor etc.) and the like, in addition to a cell established by the method including introduction of the above-mentioned genes.

Examples of the hematopoietic progenitor cell induced from a pluripotent stem cell in the present invention include a cell contained in an embryoid body or a sac-like structure and the like, obtained by culturing iPS cells or ES cells under conditions suitable for inducing differentiation into hematopoietic cells. As used herein, the "embryoid body" is a cell aggregate having a cystic structure and obtained by removing factors and feeder cells for maintaining the undifferentiated state of iPS cells and ES cells, and subjecting the iPS cells and ES cells to suspension culture (reference document: Blood, 2003, 102, 906-915). The "sac-like structure" refers to a steric cystic (having a space inside) structure derived from iPS cells or ES cells, which is formed by an endothelial cell population and the like and contains blood progenitor cells in the inside. For the detail of the sac-like structure, for example, TAKAYAMA et al., BLOOD 2008, 111: 5298-5306 can be referred to. Besides these, there is also a report on the promoted induction of hematopoietic progenitor cells from pluripotent stem cells by coculture with stromal cells (reference document: WO 2001/34776). Also, it has been reported that a cell line is established from hematopoietic progenitor cells prepared from pluripotent stem cells by culturing for a long term, and that the cell proliferation capacity thereof can be enhanced by introduction of oncogene and the like (reference document: WO 2011/034073, PLoS ONE 2008, 3:e1544).

In the present invention, differentiation of hematopoietic stem cell and/or hematopoietic progenitor cell refers to conversion of a hematopoietic stem cell to a hematopoietic progenitor cell, a pluripotent hematopoietic progenitor cell to a unipotent hematopoietic progenitor cell, a hematopoietic progenitor cell to a cell having a specific function, i.e., a mature blood cell such as erythrocyte, leukocyte, megakaryocyte and the like. The medium composition of the present invention acts on hematopoietic stem cells and/or hematopoietic progenitor cells and exhibits an effect of supporting differentiation into erythrocytes when the hematopoietic stem cells and/or hematopoietic progenitor cells are cultured ex vivo. Therefore, when hematopoietic stem cells are cultured in the medium composition of the present invention, differentiation of the hematopoietic stem cells into erythrocytes or progenitor cells thereof is promoted. Specifically, a large number of erythrocytes can be prepared ex vivo by culturing hematopoietic stem cells and/or hematopoietic progenitor cells in the medium composition. In that case, differentiation into erythrocytes can also be further promoted efficiently by further adding various cytokines and growth factors to the medium composition, coculturing with stromal cells, or further adding other low-molecular-weight compound(s) that act on the hematopoietic stem cells and/or the hematopoietic progenitor cells. The present invention also provides a culture preparation of hematopoietic stem cells and/or hematopoietic progenitor cells, which is obtained by culturing the hematopoietic stem cells and/or the hematopoietic progenitor cells in the medium composition of the present invention. The culture preparation comprises hematopoietic stem cells and/or hematopoietic progenitor cells, as well as the medium composition of the present invention.

While a culture container used for culturing hematopoietic stem cells and/or a hematopoietic progenitor cells is not particularly limited as long as it enables animal cell culture generally, for example, flask, dish, petri dish, dish for tissue culture, multidish, microplate, microwell plate, multiplate, multiwell plate, chamber slide, schale, tube, tray, culture bag, roller bottle and the like can be mentioned. While the materials of these culture tools are not particularly limited, for

example, glass, plastics such as polyvinyl chloride, cellulosic polymer, polystyrene, polymethylmethacrylate, polycarbonate, polysulfone, polyurethane, polyester, polyamide, polystyrene, polypropylene and the like can be mentioned.

As the medium to be used in the present invention, any medium used for culturing hematopoietic stem cells and/or hematopoietic progenitor cells can be used. Examples of the medium include Dulbecco's Modified Eagle's Medium (DMEM), HamF12 medium (Ham's Nutrient Mixture F12), DMEM/F12 medium, McCoy's 5A medium, Eagle MEM medium (Eagle's Minimum Essential Medium; EMEM), αMEM medium (alpha Modified Eagle's Minimum Essential Medium; αMEM), MEM medium (Minimum Essential Medium), RPMI1640 medium, Iscove's Modified Dulbecco's Medium (IMDM), StemPro34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by STEMCELL Technologies), StemSpanSFEM (manufactured by STEMCELL Technologies), StemlineII (manufactured by Sigma Aldrich), QBSF-60 (manufactured by Qualitybiological), and the like can be mentioned. In addition, for culture and passage of pluripotent stem cells, a medium generally used for maintaining pluripotent stem cells can be used. For example, DMEM/F12 medium, Iscove's Modified Dulbecco's Medium (IMDM), Dulbecco's modified Eagle medium (DMEM), HamF-12 medium, X-VIVO 10 (manufactured by Lonza), X-VIVO 15 (manufactured by Lonza), mTeSR (manufactured by STEMCELL Technologies), TeSR2 (manufactured by STEMCELL Technologies), StemProhESC SFM (manufactured by Invitrogen) and the like can be mentioned. These media can contain a cell adhesion factor, and examples thereof include Matrigel, collagen gel, gelatin, poly-L-lysine, poly-D-lysine, laminin and fibronectin. It is also possible to add two or more kinds of these cell adhesion factors in combination. Furthermore, the above-mentioned medium can be further mixed with a thickener such as guargum, tamarind gum, alginic acid propylene glycol ester, locust bean gum, gum arabic, tara gum, tamarind gum, methylcellulose and the like.

Those of ordinary skill in the art can freely add, according to the object, sodium, potassium, calcium, magnesium, phosphorus, chlorine, various amino acids, various vitamins, antibiotic, serum, fatty acid, sugar and the like to the above-mentioned medium. For culture of hematopoietic stem cells and/or hematopoietic progenitor cells, those of ordinary skill in the art can also add, according to the object, one or more kinds of other chemical components and biogenic substances in combination. Examples of the components to be added to a medium for hematopoietic stem cells and/or hematopoietic progenitor cells include fetal bovine serum, human serum, horse serum, insulin, transferrin, lactoferrin, cholesterol, ethanolamine, sodium selenite, monothioglycerol, 2-mercaptoethanol, bovine serum albumin, sodium pyruvate, polyethylene glycol, various vitamins, various amino acids, agar, agarose, collagen, methylcellulose, various cytokines, various hormones, various growth factors, various extracellular matrices, various cell adhesion molecules and the like. Examples of the cytokine to be added to a medium include, but are not limited to, interleukin-1 (IL-1), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), interleukin-13 (IL-13), interleukin-14 (IL-14), interleukin-15 (IL-15), interleukin-18 (IL-18), interleukin-21 (IL-21), interferon-α (IFN-α), interferon-β (IFN-β), interferon-γ (IFN-γ), granulocyte colony stimulating factor (G-CSF), monocyte colony stimulating agent (M-CSF), granulocyte-macrophage colony stimulating agent (GM-CSF), stem cell factor (SCF), flk2/flt3 ligand (FL), leukemia cell inhibitory factor (LIF), oncostatin M (OM), erythropoietin (EPO), thrombopoietin (TPO) and the like.

Examples of the hormone to be added to a medium include, but are not limited to, melatonin, serotonin, thyroxine, triiodothyronine, epinephrine, norepinephrine, dopamine, anti-Mullerian hormone, adiponectin, adrenocorticotropic hormone, angiotensinogen and angiotensin, antidiuretic hormone, atrial natriuretic peptide, calcitonin, cholecystokinin, corticotropin release hormone, erythropoietin, follicle stimulating hormone, gastrin, ghrelin, glucagon, gonadotropin release hormone, growth hormone release hormone, human chorionic gonadotropin, human placental lactogen, growth hormone, inhibin, insulin, insulin-like growth factor, leptin, luteinizing hormone, melanocyte stimulating hormone, oxytocin, parathyroid hormone, prolactin, secretin, somatostatin, thrombopoietin, thyroid-stimulating hormone, thyrotropin releasing hormone, cortisol, aldosterone, testosterone, dehydroepiandrosterone, androstenedione, dihydrotestosterone, estradiol, estrone, estriol, progesterone, calcitriol, calcidiol, prostaglandin, leukotriene, prostacyclin, thromboxane, prolactin releasing hormone, lipotropin, brain natriuretic peptide, neuropeptide Y, histamine, endothelin, pancreas polypeptide, rennin and enkephalin.

Examples of the growth factor to be added to a medium include, but are not limited to, transforming growth factor-α (TGF-α), transforming growth factor-β (TGF-β), macrophage inflammatory protein-1α (MIP-1α), epithelial growth factor (EGF), fibroblast growth factor-1, 2, 3, 4, 5, 6, 7, 8 or 9 (FGF-1, 2, 3, 4, 5, 6, 7, 8, 9), nerve growth factor (NGF), hepatocyte growth factor (HGF), leukemia inhibitory factor (LIF), protease nexin I, protease nexin II, platelet-derived growth factor (PDGF), cholinergic differentiation factor (CDF), various chemokines, Notch ligand (Delta 1 and the like), Wnt protein, angiopoietin-like protein-2, 3, 5 or 7 (Angpt 2, 3, 5, 7), insulin-like growth factor (IGF), insulin-like growth factor binding protein-1 (IGFBP), pleiotrophin and the like.

In addition, these cytokines and growth factors having amino acid sequences artificially altered by gene recombinant techniques can also be added. Examples thereof include IL-6/soluble IL-6 receptor complex, Hyper IL-6 (fusion protein of IL-6 and soluble IL-6 receptor) and the like.

Examples of the various extracellular matrices and various cell adhesion molecules include collagen I to XIX,

fibronectin, vitronectin, laminin-1 to 12, nitogen, tenascin, thrombospondin, von Willebrand factor, osteopontin, fibrinogen, various elastins, various proteoglycans, various cadherins, desmocolin, desmoglein, various integrins, E-selectin, P-selectin, L-selectin, immunoglobulin superfamily, Matrigel, poly-D-lysine, poly-L-lysine, chitin, chitosan, sepharose, hyaluronic acid, alginate gel, various hydrogels, cleavage fragments thereof and the like.

To achieve induction of differentiation of hematopoietic stem cells and/or hematopoietic progenitor cells into erythrocytes, the medium composition of the present invention preferably contains one or two or more factors selected from the group consisting of stem cell factor (SCF), interleukin-3 (IL-3), interleukin-6 (IL-6), interleukin-11 (IL-11), flk2/flt3 ligand (FL), thrombopoietin (TPO) and erythropoietin (EPO), which are known as factors that induce differentiation into erythrocytes among the above-mentioned cytokines and growth factors. The medium composition of the present invention more preferably contains one or two or more factors selected from the group consisting of stem cell factor (SCF), flk2/flt3 ligand (FL), interleukin-3 (IL-3), thrombopoietin (TPO) and erythropoietin (EPO), and most preferably contains 1, 2 or 3 factors selected from the group consisting of SCF, IL-3 and EPO. The concentration of cytokines and growth factors to be added during culture can be appropriately set within the range where induction of differentiation of hematopoietic stem cells and/or hematopoietic progenitor cells into erythrocytes can be achieved, and is generally 0.1 ng/mL to 1000 ng/mL, preferably 1 ng/mL to 100 ng/mL.

The above-mentioned chemical components and biogenic substances can be used not only by addition to a medium, but also by immobilizing on a surface of basal plate or carrier during culture. To be specific, it can be achieved by dissolving the object component in an appropriate solvent, coating the surface of basal plate or carrier with the same, and washing away excess components. Alternatively, the surface of basal plate or carrier may be coated in advance with a substance that specifically binds to the object component, and the object component may be added onto the basal plate.

Examples of the antibiotic to be added to a medium include sulfa drugs, penicillin, phenethicillin, methicillin, oxacillin, cloxacillin, dicloxacillin, flucloxacillin, nafcillin, ampicillin, penicillin, amoxicillin, ciclacillin, carbenicillin, ticarcillin, piperacillin, azlocillin, mezlocillin, mecillinam, andinocillin, cephalosporin and a derivative thereof, oxolinic acid, amifloxacin, temafloxacin, nalidixic acid, piromidic acid, ciprofloxacin, cinoxacin, norfloxacin, perfloxacin, rosaxacin, ofloxacin, enoxacin, pipemidic acid, sulbactam, clavulanic acid, β-bromopenisillanic acid, β-chloropenisillanic acid, 6-acetylmethylene-penisillanic acid, cephoxazole, sultampicillin, formaldehyde hydrate esters of amdinocillin and sulbactam, tazobactam, aztreonam, sulfazethin, isosulfazethin, norcardicin, m-carboxyphenyl, phenylacetamidophosphonic acid methyl, chlortetracycline, oxytetracycline, tetracycline, demeclocycline, doxycycline, methacycline, and minocycline.

The culture temperature of hematopoietic stem cells and/or hematopoietic progenitor cells is generally 25 to 39°C, preferably 33 to 39°C. The $CO_2$ concentration is generally 4 to 10% by volume, preferably 4 to 6% by volume, in the culture atmosphere. The culture period can be generally set to be 3 to 120 days, preferably 7 to 35 days, more preferably 14 to 21 days.

When hematopoietic stem cells and/or a hematopoietic progenitor cells are cocultured with stromal cells in the method of the present invention, the coculture can be performed by collecting bone marrow cells and directly culturing them. The coculture can also be performed by collecting bone marrow, isolating stromal cells, hematopoietic stem cells and/or hematopoietic progenitor cells, and other cell population and the like, combining stromal cells of an individual other than the one from whom the bone marrow was collected and the hematopoietic stem cells and/or the hematopoietic progenitor cells. In addition, the coculture can be performed by culturing stromal cells alone to grow and then adding hematopoietic stem cells and/or hematopoietic progenitor cells thereto. As the culture conditions and medium composition therefor, those described above can be used. As the stromal cell, any cell can be used as long as it contributes to the growth and maintenance of hematopoietic stem cells and/or hematopoietic progenitor cells and, for example, mouse embryonic fibroblast (MEF), SL10 cell, preferably, C3H10T1/2 cell line, OP9 cell, ST2 cell, NIH3T3 cell, PA6 cell, M15 cell, human mesenchymal stem cell (MSC), human umbilical vein endothelial cell (HUVEC), human endometrium epithelial cell and the like, more preferably C3H10T1/2 cell line, OP9 cell, and human mesenchymal stem cell (MSC) can be used. When a stromal cell is used, for example, the cell growth can also be suppressed by a mitomycin C treatment, radiation and the like.

The hematopoietic stem cells and/or hematopoietic progenitor cells can also be cultured by automatically conducting cell seeding, medium exchange, cell image obtainment, and recovery of cultured cells, under a mechanical control and under a closed environment while controlling pH, temperature, oxygen concentration and the like and using a bioreactor and an automatic incubator capable of high density culture. As a method for supplying a fresh medium and feeding the required substances to the cells and/or tissues during the culture using such apparatuses, fed-batch culture, continuous culture and perfusion culture are available, and all these methods can be used for the culture method of the present invention.

The particular compound to be used in the present invention promotes differentiation of hematopoietic stem cells and/or hematopoietic progenitor cells into erythrocytes (preferably, differentiation of hematopoietic stem cell and/or hematopoietic progenitor cell into erythrocyte by the aforementioned factors which induce differentiation into erythrocytes). The particular compound to be used in the present invention promotes differentiation of hematopoietic stem cells into erythrocytes or progenitor cells thereof (preferably, differentiation of hematopoietic stem cells into erythrocytes or progenitor cells thereof by the aforementioned factors which induce differentiation into erythrocyte).

Examples of the particular compound to be used in the present invention include, but are not limited to, polymer compounds, preferably a polymer compound having an anionic functional group.

As the anionic functional group, carboxylic acid, sulfonic acid, phosphoric acid and a salt thereof can be mentioned, with preference given to carboxylic acid or a salt thereof.

As a polymer compound to be used in the present invention, one constituted of one or two or more kinds selected from the aforementioned anionic functional groups can be used.

Specific preferable examples of the polymer compound to be used in the present invention include, but are not limited to, polysaccharides wherein not less than 10 monosaccharides (e.g., triose, tetrose, pentose, hexsauce, heptose etc.) are polymerized, more preferably, acidic polysaccharides having an anionic functional group. The acidic polysaccharides here is not particularly limited as long as it has an anionic functional group in the structure thereof, and includes, for example, polysaccharides having a uronic acid (e.g., glucuronic acid, iduronic acid, galacturonic acid, mannuronic acid), polysaccharides having a sulfuric acid or phosphoric acid in a part of the structure thereof, and polysaccharides having the both structures, and includes not only naturally-obtained polysaccharides but also polysaccharides produced by microorganisms, polysaccharides produced by genetic engineering, and polysaccharides artificially synthesized using an enzyme. More specifically, examples thereof include polymer compounds composed of one or two or more kinds selected from the group consisting of hyaluronic acid, gellan gum, deacylated gellan gum, rhamsan gum, diutan gum, xanthan gum, carageenan, xanthan gum, hexuronic acid, alginic acid, fucoidan, pectin, pectic acid, pectinic acid, heparan sulfate, heparin, heparitin sulfate, keratosulfate, chondroitin sulfate, dermatan sulfate, rhamnan sulfate and a salt thereof.

The salt here includes, for example, salts with alkali metal such as lithium, sodium, potassium; salts with alkaline earth metals such as calcium, barium, magnesium; and salts with aluminum, zinc, copper, iron, ammonium, organic base and amino acid and the like.

The weight average molecular weight of these polymer compounds or polysaccharides is preferably 10,000 to 50,000,000, more preferably 100,000 to 20,000,000, still more preferably 1,000,000 to 10,000,000. For example, the molecular weight can be measured based on pullulan by gel penetration chromatography (GPC) .

More specific preferable examples of the particular compound to be used in the present invention include hyaluronic acid, deacylated gellan gum, diutan gum, carageenan and xanthan gum and a salt thereof. Most preferable examples include deacylated gellan gum and a salt thereof, since the viscosity of the medium composition can be made low and the cells or tissues can be easily recovered.

The deacylated gellan gum in the present invention is a linear high molecular weight polysaccharide containing 4 molecules of sugars of 1-3 bonded glucose, 1-4 bonded glucuronic acid, 1-4 bonded glucose and 1-4 bonded rhamnose as the constituent unit, which is a polysaccharide of the following formula (I) wherein R1, R2 are each a hydrogen atom, and n is an integer of two or more. R1 may contain a glyceryl group, R2 may contain an acetyl group, and the content of the acetyl group and glyceryl group is preferably not more than 10%, more preferably not more than 1%.

The particular compound of the present invention takes various forms when added to a liquid medium. In the case of deacylated gellan gum, it uptakes a metal ion (e.g., calcium ion) in a liquid medium when mixed with the liquid medium, forms an indeterminate structure via the metal ion. The viscosity of the medium composition of the present invention prepared from deacylated gellan gum is not more than 8 mPa·s, preferably not more than 4 mPa·s, and more preferably not more than 2 mPa·s for easy recovery of the cells or tissues.

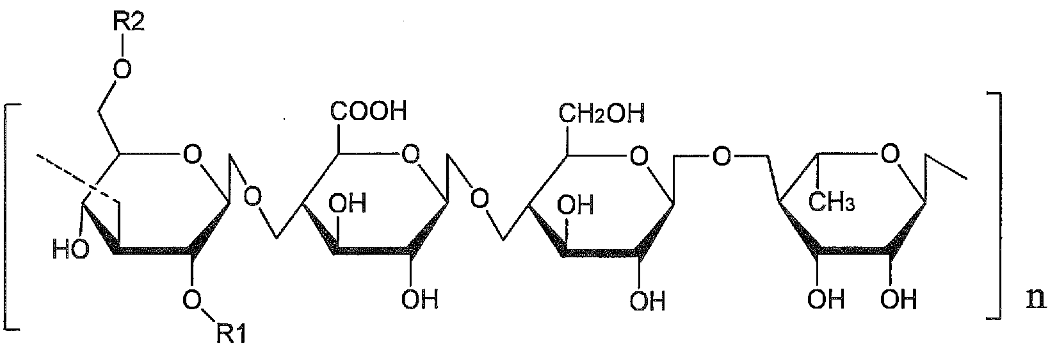

(I)

The particular compound in the present invention can be obtained by a chemical synthesis method. When the compound is a naturally-occurring substance, it is preferably obtained from various plants, various animals, various microorganisms containing the compound by extraction, separation and purification by conventional techniques. For extraction, the compound can be extracted efficiently by using water and supercritical gas. For example, as a production

method of gellan gum, producing microorganisms are cultured in a fermentation medium, mucous products produced outside the bacterial cells are recovered by a general purification method, and, after the processes of drying, pulverizing and the like, the products are powderized. In the case of deacylated gellan gum, an alkali treatment should be applied when the mucous products are recovered, to deacylate the glyceryl group and the acetyl group bonded to 1-3 bonded glucose residue, and then the given products should be recovered. Examples of the purification method include liquid-liquid extraction, fractional precipitation, crystallization, various kinds of ion exchange chromatography, gel filtration chromatography using Sephadex LH-20 and the like, adsorption chromatography using activated carbon, silica gel and the like, adsorption and desorption treatment of active substance by thin layer chromatography, high performance liquid chromatography using reversed-phase column and the like, and impurity can be removed and the compound can be purified by using them singly or in combination in any order, or repeatedly.

Examples of the gellan gum-producing microorganism include, but are not limited to, Sphingomonas elodea and microorganism obtained by altering the gene of Sphingomonas elodea.

In the case of deacylated gellan gum, commercially available products, for example, "KELCAOGEL (registered trade mark of CP Kelco) CG-LA" manufactured by SANSHO Co., Ltd., "KELCOGEL (registered trade mark of CP Kelco)" manufactured by San-Ei Gen F.F.I., Inc. and the like can be used. [0038]

The concentration of particular compound in the medium can be appropriately set within the range where differentiation of hematopoietic stem cells and/or hematopoietic progenitor cells into erythrocytes can be promoted, and is generally 0.0005% to 1.0% (weight/volume), preferably 0.001% to 0.4% (weight/volume), more preferably 0.005% to 0.1% (weight/volume), still more preferably 0.005% to 0.05% (weight/volume). For example, in the case of deacylated gellan gum, it can be added to a medium at generally 0.001 to 1.0, preferably 0.003 to 0.5, more preferably 0.005 to 0.1, most preferably, 0.015 to 0.03% (weight/volume).

The concentration can be calculated by the following formula.

$$\text{Concentration (\%)} = \text{weight (g) of particular compound/volume (ml) of medium composition} \times 100$$

The aforementioned compound can also be further converted to a different derivative by a chemical synthesis method, and the thus-obtained derivative can also be used effectively in the present invention. Specifically, in the case of deacylated gellan gum, a derivative of a compound represented by the formula (I) wherein a hydroxyl group for R1 and/or R2 is substituted by $C_{1-3}$ alkoxy group, $C_{1-3}$ alkylsulfonyl group, a monosaccharide residue such as glucose, fructose and the like, oligosaccharide residue such as sucrose, lactose and the like, amino acid residue such as glycine, arginine and the like can also be used in the present invention. In addition, the compound can also be crosslinked using a crosslinking agent such as 1-ethyl-3-(3-di-methylaminopropyl)carbodiimide (EDC) and the like.

The particular compound or a salt thereof to be used in the present invention can be present in any crystal form depending on the production conditions, and can be present as any hydrate. Such crystal form, hydrate and mixtures thereof are also encompassed in the scope of the present invention. In addition, they may be present as a solvate containing an organic solvent such as acetone, ethanol, tetrahydrofuran and the like. Such forms are all encompassed in the scope of the present invention.

The particular compound to be used in the present invention may be present in the form of tautomer formed by isomerization in the ring or outside the ring, geometric isomer or tautomer, or a mixture of geometric isomers, or mixtures thereof. When the compound of the present invention has an asymmetric center, irrespective of whether the compound is formed by isomerization, it may be present in the form of a resolved optical isomer or a mixture containing same at any ratio.

The medium composition of the present invention may contain a metal ion, for example, a divalent metal ion (calcium ion, magnesium ion, zinc ion, ferrous ion, copper ion etc.), and preferably contains calcium ion.

When the particular compound in the present invention is added to the above-mentioned medium, the particular compound is dissolved or dispersed in an appropriate solvent when in use (this is used as a medium additive). Thereafter, the medium additives can be added to a medium such that the concentration of the particular compound in the medium promotes differentiation of hematopoietic stem cell and/or hematopoietic progenitor cell into erythrocyte (generally 0.0005% to 1.0% (weight/volume), preferably 0.001% to 0.4% (weight/volume), more preferably 0.005% to 0.1% (weight/volume), further preferably 0.005% to 0.05% (weight/volume)). In the case of a deacylated gellan gum, it can be added to a medium generally at 0.001 to 1.0, preferably 0.003 to 0.5, more preferably 0.005 to 0.1, most preferably 0.015 to 0.03% (weight/volume).

The concentration can be calculated by the following formula.

$$\text{Concentration (\%)} = \text{weight (g) of particular compound/volume (ml) of medium composition} \times 100$$

The above-mentioned medium additives are used for promoting differentiation of hematopoietic stem cells and/or hematopoietic progenitor cells into erythrocytes (preferably, differentiation of hematopoietic stem cells and/or hematopoietic progenitor cells into erythrocytes by the aforementioned factors which induce differentiation into erythrocytes). The above-mentioned medium additives are used for promoting differentiation of hematopoietic stem cells into erythrocytes or progenitor cells thereof (preferably, differentiation of hematopoietic stem cells into erythrocytes or progenitor cells thereof by the aforementioned factors which induces differentiation into erythrocyte).

Here, examples of appropriate solvent used for the medium additive include, but are not limited to, aqueous solvents such as water, dimethyl sulfoxide (DMSO), various alcohols (e.g., methanol, ethanol, butanol, propanol, glycerol, propylene glycol, butyleneglycol and the like), and the like. In this case, the concentration of the particular compound is 0.001% to 5.0% (weight/volume), preferably 0.01% to 1.0% (weight/volume), more preferably 0.1% to 0.5% (weight/volume). It is also possible to further add an additive to enhance the effect of the particular compound, or lower the concentration when in use. As an example of such additive, one or more kinds of polysaccharides including guargum, tamarind gum, alginic acid propylene glycol ester, locust bean gum, gum arabic, tara gum, tamarind gum, methylcellulose and the like can be mixed. It is also possible to immobilize the particular compound on the surface of a carrier or make the particular compound be supported inside a carrier during culture. The particular compound can have any form during provision or preservation. The particular compound may be in the form of a formulated solid such as tablet, pill, capsule, granule, or a liquid such as a solution obtained by dissolving in an appropriate solvent using a solubilizer or a suspension, or may be bonded to a basal plate or a single substance. Examples of the additive used for formulating include preservatives such as p-hydroxybenzoates and the like; excipients such as lactose, glucose, sucrose, mannit and the like; lubricants such as magnesium stearate, talc and the like; binders such as polyvinyl alcohol, hydroxypropylcellulose, gelatin and the like; surfactants such as fatty acid ester and the like; plasticizers such as glycerol and the like; and the like. These additives are not limited to those mentioned above, and can be selected freely as long as they are utilizable for those of ordinary skill in the art. The particular compound of the present invention may be sterilized as necessary. The sterilization method is not particularly limited, and, for example, radiation sterilization, ethylene oxide gas sterilization, autoclave sterilization and the like can be mentioned. The sterilization treatment may be applied when the particular compound is in a solid state or a solution state.

Examples of the preparation method of the medium composition of the present invention are shown below, which are not to be construed as limitative. The particular compound is added to ion-exchanged water or ultrapure water. Then, the mixture is stirred with heating at a temperature at which the particular compound can be dissolved (e.g., not less than 60°C, not less than 80°C, not less than 90°C) to allow for dissolution to a transparent state. After dissolving, the mixture is allowed to cool with stirring, and sterilized (e.g., autoclave sterilization at 121°C for 20 min). After cooling to room temperature, the aforementioned sterilized aqueous solution is added with stirring (e.g., homomixer etc.) to a given medium to be used for static culture and mix the solution with the medium to be homogeneous. The method of mixing the aqueous solution and the medium is not particularly limited, and may be manual mixing such as pipetting etc., or mixing with an instrument such as magnetic stirrer, mechanical stirrer, homomixer and homogenizer. Furthermore, the medium composition of the present invention can be filtrated through a filter after mixing. The size of the pore of the filter to be used for the filtration treatment is 5 μm to 100 μm, preferably 5 μm to 70 μm, more preferably 10 μm to 70 μm.

For example, when deacylated gellan gum is prepared, deacylated gellan gum is added to ionexchanged water or ultrapure water to 0.1% to 1% (weight/volume), preferably 0.2% to 0.5% (weight/volume), more preferably 0.3% to 0.4% (weight/volume). Then, the aforementioned deacylated gellan gum is dissolved to a transparent state by stirring with heating at any temperature as long as dissolution is possible, which may be not less than 60°C, preferably not less than 80°C, more preferably not less than 90°C. After dissolution, the mixture is allowed to cool with stirring, and sterilized with autoclave at, for example, 121°C for 20 min. After cooling to room temperature, the aqueous solution is added to, for example, IMDM medium with stirring by a homomixer and the like to a desired final concentration (e.g., when the final concentration is 0.015%, the ratio of 0.3% aqueous solution:medium is 1:20), and the mixture is homogeneously mixed. The mixing method of the aqueous solution and the medium is not particularly limited, and may be manual mixing such as pipetting etc., or mixing with an instrument such as magnetic stirrer, mechanical stirrer, homomixer and homogenizer. Furthermore, the medium composition of the present invention can be filtrated through a filter after mixing. The size of the pore of the filter to be used for the filtration treatment is 5 μm to 100 μm, preferably 5 μm to 70 μm, more preferably 10 μm to 70 μm.

Those of ordinary skill in the art can freely select the form and state of the hematopoietic stem cells and/or hematopoietic progenitor cells to be cultured by the method of the present invention. Specific preferable examples thereof include, but are not particularly limited to, a state in which the cells are singly dispersed in the medium composition, a

state in which plural cells assemble and form cell aggregates (spheres), or a state in which two or more kinds of cells assemble and form cell aggregates (spheres), and the like.

When hematopoietic stem cells and/or hematopoietic progenitor cells are cultured by the method of the present invention, hematopoietic stem cells and/or hematopoietic progenitor cells prepared separately are added to the culture composition of the present invention and mixed to be dispersed homogeneously. In this case, the mixing method is not particularly limited and, for example, manual mixing using pipetting and the like, mixing using instrument such as stirrer, vortex mixer, microplate mixer, shaker and the like can be mentioned. After mixing, the culture may be stood still, or the culture may be rotated, shaken or stirred as necessary. The rotation number and frequency can be appropriately set according to the object of those of ordinary skill in the art. When the medium composition needs to be exchanged during the static culture period, the cells and/or tissues and the medium composition should be separated by centrifugation or filtration treatment, and a fresh medium composition can be added to the cells and/or tissues. Alternatively, the cells and/or tissues should be appropriately concentrated by centrifugation or filtration treatment, and a fresh medium composition can be added to the concentrated liquid. For example, unlimitatively, the gravitational acceleration (G) of centrifugation is 100G to 400G, and the size of the pore of the filter used for the filtration treatment is 10 $\mu$m to 100 $\mu$m. In addition, using magnetic microbeads coated, on the surface, with an antibody that specifically binds to the object cell, the cultured cells and/or tissues can be isolated by magnetic force. Examples of such magnetic microbeads include Dynabeads (manufactured by Veritas Ltd.), MACS microbead (manufactured by Miltenyi Biotec), BioMag (manufactured by Techno Chemicals Corporation) and the like. Exchange of the medium composition can also be performed by using a bioreactor and an automatic incubator capable of conducting under a mechanical control and under a closed environment. The frequency of medium change is not particularly limited, and those of ordinary skill in the art can make an appropriate selection.

A preferable example of the production method of erythrocytes from hematopoietic stem cells and/or hematopoietic progenitor cells by the present invention is shown below.

First, hematopoietic stem cells and/or hematopoietic progenitor cells derived from living organism are prepared by collecting, for example, cord blood, bone marrow, peripheral blood and the like and separating a cell population rich in hematopoietic stem cells and/or hematopoietic progenitor cells therefrom. Examples of such cell population include CD34 positive cells, CD133 positive cells and the like. For example, CD34 positive cells can be isolated by a specific gravity centrifugation method and a magnetic cell separation (Magnetic Cell Sorting; MACS) system or flow cytometry in combination. For example, blood added with CPD solution (citric acid-phosphoric acid-dextran) is fractionated by a specific gravity centrifugation method and the like, and a fraction containing many mononuclear cells (hereinafter to be referred to as nucleated cell fraction) is separated and recovered. Examples of the specific gravity centrifugation method include a specific gravity centrifugation method using dextran and Ficoll solution, a Ficoll-paque density gradient method, a Percoll discontinuous density gradient specific gravity centrifugation method, a density gradient specific gravity centrifugation method using Lymphoprep and the like. Then, magnetic beads immobilized with an anti-human CD34 monoclonal antibody (manufactured by Miltenyi Biotec; hereinafter to be referred to as CD34 antibody magnetic beads) and the nucleated cell fraction separated and recovered above are mixed, and then incubated at about 2 to 8°C (about 30 min) to allow the CD34 positive cells in the nucleated cell fraction to bind to the antibody magnetic beads. The bonded antibody magnetic beads/CD34 positive cells are separated and recovered by an exclusive magnetic cell separation apparatus, for example, auto MACS system (manufactured by Miltenyi Biotec) and the like. In one embodiment, the purity of the isolated hematopoietic stem cells and/or hematopoietic progenitor cells (e.g., CD34 positive cells) (percentage of the object cell number in the total cell number) is generally not less than 70%, preferably not less than 80%, more preferably not less than 90%, further preferably not less than 99%, most preferably 100%. The thus-obtained CD34 positive cells are cultured in the culture composition of the present invention to allow for differentiation of the CD34 positive cells into erythrocytes. While the conditions, culture apparatus, the kind of medium, the kind of the compound of the present invention, the content of the compound of the present invention, the kind of the additive, the content of the additive, culture period, culture temperature, and the like for the culture of the CD34 positive cells can be appropriately selected by the artisan from the ranges described in the present specification, they are not limited thereto.

After the culture, the total number of cells is measured by a Trypan Blue method and the like, the cultured cells are stained with an anti-CD71 antibody, an anti-CD36 antibody or an anti-glycophorin A antibody labeled with a fluorescence dye such as FITC (fluorescein isothiocyanate), PE (phycoerythrin), APC (allophycocyanin) and the like, and the ratio of the cells positive to these erythrocyte-specific markers is analyzed by flow cytometry, whereby the degree of expansion of the erythrocytes in the cultured cells can be determined. In this case, the ratio of the differentiated cells can also be determined by staining with an anti-CD34 antibody. In addition, differentiation into erythrocyte may also be evaluated by visual examination of the cells under a microscope. The presence of erythrocyte can be confirmed by the presence of a typical biconcave cell. The presence of erythrocyte (including reticulocyte) can also be confirmed by staining with deoxyribonucleic acid (DNA) such as Hoechst 33342, TO-PRO (registered trade mark)-3, DRAG5 and the like. Erythrocytes and reticulocytes are generally negative to the staining with these. Moreover, the proportion of the erythroid hematopoietic progenitor cells can be determined by subjecting a portion of the culture to a colony assay and

counting the number of erythrocyte colonies formed. Erythrocytes produced by the above-mentioned methods can be separated from hematopoietic stem cells and/or hematopoietic progenitor cells. The separation can be achieved by using, for example, an antibody to CD36 and/or glycophorin A. Examples of the isolation method include magnetic bead separation via an antibody, cell sorting, passage of cells through a membrane or column conjugated with an antibody to CD36 and/or glycophorin A and the like. In addition, hematopoietic stem cells and/or hematopoietic progenitor cells in the culture can be killed by UV radiation. Example

The present invention is explained in more detail in the following by specifically describing an experimental example using the medium composition of the present invention as an Example, which is not to be construed as limitative.

[Experimental Example]

The $CO_2$ concentration (%) in a $CO_2$ incubator is shown by % volume of $CO_2$ in the atmosphere. PBS means phosphate buffered saline (manufactured by Sigma Aldrich Japan), and FBS means fetal bovine serum (manufactured by Biological Industries). In addition, (w/v) indicates weight per volume.

(Experimental Example 1: Expansion experiment of erythrocyte progenitor cells using human cord blood CD34 positive cells)

Deacylated gellan gum (KELCOGEL CG-LA, manufactured by SANSHO Co., Ltd.) was suspended in ultrapure water (Milli-Q water) to 0.3%(w/v), and dissolved by stirring with heating at 90°C. This aqueous solution was sterilized at 121°C for 20 min in an autoclave. Using this solution, a medium composition was prepared by adding deacylated gellan gum at a final concentration of 0.015%(w/v) or 0.030%(w/v) to StemSpanSFEM (manufactured by STEMCELL Technologies) added with SCF (manufactured by Wako Pure Chemical Industries, Ltd.) at a final concentration of 100 ng/mL, IL-3 (manufactured by Wako Pure Chemical Industries, Ltd.) at a final concentration of 20 ng/mL and EPO (manufactured by Mitsubishi Tanabe Pharma) at a final concentration of 1 unit/mL. Successively, CD34 positive cells of human cord blood purchased from Lonza were inoculated to the above-mentioned medium composition added with deacylated gellan gum at 100000 cells/mL, and dispensed to wells of a 24-well plate (manufactured by Corning Incorporated) at 1 mL/well. As a negative control, suspension of CD34 positive cells in the above medium free of deacylated gellan gum was dispensed.

Successively, this plate was cultured in a $CO_2$ incubator (37°C, 5% $CO_2$) for 7 days in a standing state, and the number of viable cells was measured by a Trypan Blue method. The number of glycophorin A positive CD34 negative cells was calculated as follows. First, the cells after liquid culture were stained with an anti-glycophorin A antibody (APC, manufactured by Becton, Dickinson and Company) and an anti-CD34 antibody (PE, manufactured by Becton, Dickinson and Company). The stained cells were washed with PBS(-) solution containing 2%(v/v) FBS, and stained with propidium iodide (manufactured by Sigma-Aldrich Japan) added at a final concentration of 1 μg/mL. The stained cells were analyzed by BD FACSAria™ (registered trade mark) III flow cytometer (manufactured by Becton, Dickinson and Company), the ratio of glycophorin A-positive CD34-negative cells was determined, and the number of viable cells was multiplied by the ratio, whereby the number of glycophorin A-positive CD34-negative cells was calculated.

As a result, the medium composition of the present invention showed a superior expanding activity on glycophorin-A positive CD34-negative cells, and was confirmed to have an activity to expand erythrocyte progenitor cells. The expansion ratio on addition of 0.015% or 0.030% deacylated gellan gum when the number of glycophorin-A positive CD34-negative cells without addition of deacylated gellan gum is 1 is shown in Table 1.

Table 1

| deacylated gellan gum concentration (%) | no addition | 0.015 | 0.030 |
|---|---|---|---|
| relative glycophorin-A positive CD34-negative cell number | 1.00 | 1.75 | 1.85 |

Industrial Applicability

The medium composition of the present invention is extremely useful for ex vivo production of erythrocytes from hematopoietic stem cells and/or hematopoietic progenitor cells and the like. The erythrocytes produced by the method of the present invention are extremely useful for a medical treatment requiring erythrocyte transfusion and the like.

All references cited in the present specification, including publication, patent document and the like, are hereby

incorporated individually and specifically by reference, to the extent that the entireties thereof have been specifically disclosed herein.

This application is based on a patent application No. 2013-086904 filed in Japan (filing date: April 17, 2013), the contents of which are incorporated in full herein.

## Claims

1. A medium additive comprising a polysaccharide, which is used for differentiating hematopoietic stem cells and/or hematopoietic progenitor cells into erythrocytes.

2. The additive according to claim 1, wherein said polysaccharide has an anionic functional group.

3. The additive according to claim 2, wherein said anionic functional group is at least one selected from the group consisting of carboxyl group, sulfo group and phosphate group.

4. The additive according to claim 3, wherein said polysaccharide is at least one selected from the group consisting of hyaluronic acid, gellan gum, deacylated gellan gum, xanthan gum, carageenan, diutan gum, alginic acid, fucoidan, pectin, pectic acid, pectinic acid, heparan sulfate, heparin, heparitin sulfate, keratosulfate, chondroitin sulfate, dermatan sulfate, rhamnan sulfate and salts thereof.

5. The additive according to claim 4, wherein said polysaccharide is deacylated gellan gum or a salt thereof.

6. A medium composition for erythrocyte differentiation, which comprises the additive according to any one of claims 1 to 5.

7. The medium composition according to claim 6, further comprising one or two or more factors selected from the group consisting of SCF, IL-3, IL-6, IL-11, FL, TPO and EPO.

8. A method of producing erythrocytes from hematopoietic stem cells and/or hematopoietic progenitor cells, comprising culturing the hematopoietic stem cells and/or the hematopoietic progenitor cells in the presence of the additive according to any one of claims 1 to 5, or in the medium composition according to claim 6 or 7.

9. A method of inducing differentiation of hematopoietic stem cells into erythrocytes or progenitor cells thereof, comprising culturing the hematopoietic stem cells in the presence of the additive according to any one of claims 1 to 5, or in the medium composition according to claim 6 or 7.

10. A culture preparation of hematopoietic stem cells and/or hematopoietic progenitor cells, comprising hematopoietic stem cells and/or hematopoietic progenitor cells, and the medium composition according to claim 6 or 7.

**INTERNATIONAL SEARCH REPORT**

International application No.
PCT/JP2014/060829

A. CLASSIFICATION OF SUBJECT MATTER
*C12N1/00*(2006.01)i, *C12N5/0789*(2010.01)i

According to International Patent Classification (IPC) or to both national classification and IPC

B. FIELDS SEARCHED

Minimum documentation searched (classification system followed by classification symbols)
C12N1/00, C12N5/0789

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | JP 2010-525836 A (Whitehead Institute for Biomedical Research), 29 July 2010 (29.07.2010), claims; paragraphs [0019], [0066]; examples & US 2011/0117061 A1 & EP 2155862 A & WO 2008/137641 A2 | 1-4,6-10 |
| X | WO 2006/090886 A1 (Foundation for Biomedical Research and Innovation), 31 August 2006 (31.08.2006), claims; paragraph [0005]; examples & US 2008/0166751 A1 & EP 1860196 A1 | 1-4,6-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 June, 2014 (23.06.14) | 01 July, 2014 (01.07.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2014/060829

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 2014/017513 A1 (Nissan Chemical Industries, Ltd.), 30 January 2014 (30.01.2014), claims; paragraphs [0020], [0034] to [0046], [0055]; examples (Family: none) | 1-10 |
| A | JP 2011-24423 A (Masayuki ISHIHARA), 10 February 2011 (10.02.2011), claims; paragraphs [0016] to [0023]; examples & WO 2009/066468 A1 | 1-10 |
| A | Kishimoto S. et al., Cytokine-immobilized microparticle-coated plates for culturing hematopoetic progenitor cells, Journal of Controlled Release, 2009, Vol.133, pp.185-190 | 1-10 |
| P,A | Ayako OTANI et al., "Kobunshi Zairyo o Mochiita Shinki Sanjigen Baiyoho no Kaihatsu", Dai 36 Kai The Molecular Biology Society of Japan Nenkai Koen Yoshishu, 20 November 2013 (20.11. 2013), 2P-0990 | 1-10 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009137629 A
- WO 2010098079 A
- WO 200134776 A
- WO 2011034073 A
- JP 2013086904 A


### Non-patent literature cited in the description


- **MOUNTFORD et al.** *British Journal of Haematology,* 2010, vol. 149, 22-34
- **DOLZNIG et al.** *Curr. Biol.,* 2002, vol. 12, 1076-1085
- **GIARRATANA et al.** *Blood,* 2011, vol. 118, 5071-5079
- **MA et al.** *Proceedings of the National Academy of Sciences,* 2008, vol. 105, 13087-13092
- **DIAS et al.** *Stem Cells and Development,* 2011, vol. 20, 1639-1647
- **FUJIMI et al.** *International Journal of Hematology,* 2008, vol. 87, 339-350
- **HIROYAMA et al.** *PLoS ONE,* 2008, vol. 3, e1544
- *Nature,* 2008, vol. 456, 344-349
- *Blood,* 2003, vol. 102, 906-915
- **TAKAYAMA et al.** *BLOOD,* 2008, vol. 111, 5298-5306
- *PLoS ONE,* 2008, vol. 3, e1544